# EUROPEAN PATENT APPLICATION

(11) **EP 4 474 008 A1**
(43) Date of publication of application: **11.12.2024**
(21) Application number: 23774223.4
(22) Date of filing: 25.01.2023
(51) Int. Cl.: A61N 5/10

(54) **ELECTRON BEAM RADIATION DEVICE AND ELECTRON BEAM RADIATION METHOD**

(30) Priority: 23.03.2022 JP 2022046567
(71) Applicant: OSAKA UNIVERSITY, Suita-shi Osaka 565-0871 (JP)
(72) Inventor: HOSOKAI, Tomonao, Suita-shi, Osaka 565-0871 (JP); YAMASHITA, Yasunobu, Suita-shi, Osaka 565-0871 (JP); MUROYA, Yusa, Suita-shi, Osaka 565-0871 (JP); SUZUKI, Takayoshi, Suita-shi, Osaka 565-0871 (JP)
(74) Representative: Berggren Oy
(86) International application number: PCT/JP2023/002326
(87) International publication number: WO 2023/181623

(57) **Abstract**

An electron beam irradiation device includes an arrangement unit that arranges an irradiation target containing a prodrug therein, and an irradiation unit configured to irradiate the prodrug inside the irradiation target arranged in the arrangement unit with an electron beam having an energy higher than 1 MeV to change the prodrug into an active substance.

## Description

### Technical Field

One aspect of the present invention relates to an electron beam irradiation device and an electron beam irradiation method.

### Background Art

A prodrug is, for example, a drug that reaches a target site in the body and is then changed to an active substance by endogenous and exogenous triggers, and can express an activity at an aimed part. A technique using an oxidative environment or an acidic environment, a technique using an azoreductase derived from intestinal bacteria, and the like have been known as the endogenous trigger. A technique using light irradiation, a technique utilizing a metal catalyst or a biocatalyst, and the like have been known as the exogenous trigger.

However, in these techniques, the expression of the activity of the prodrug often occurs at a site other than the aimed part. Therefore, for example, as described in Non Patent Literature 1, a technique in which a prodrug is irradiated with X-rays, an irradiation site is changed to an active substance, and the active substance is expressed has been developed.

### Citation List

### Non Patent Literature

Non Patent Literature 1: Jin Geng, 9 others, "Switching on prodrugs using radiotherapy", Nature Chemistry, VOL 13, AUGUST 2021, 805-810

### Summary of Invention

### Technical Problem

In a case where the prodrug is present in a deep part of an irradiation target, since X-ray has large energy attenuation in a substance, the X-rays hardly reach the prodrug. Thus, the above-described technique can be applied in a case where the activity of the prodrug is expressed in the vicinity of a surface of the irradiation target, but is hardly applied in a case where the activity of the prodrug is expressed in the deep part of the irradiation target.

An object of an aspect of the present invention is to provide an electron beam irradiation device and an electron beam irradiation method capable of expressing activity of a prodrug even in a deep part of an irradiation target.

### Solution to Problem

As a result of intensive studies, the present inventors have found that a high energy electron beam can act as a trigger to change the prodrug into an active substance by irradiating a prodrug with a high energy electron beam. Then, the present inventors have found that a high energy electron beam can sufficiently reach a prodrug present in a deep part of an irradiation target because of low energy attenuation in a substance, and have completed the aspect of the present invention.

That is, an electron beam irradiation device according to an aspect of the present invention includes an arrangement unit that arranges an irradiation target containing a prodrug therein, and an irradiation unit configured to irradiate the prodrug inside the irradiation target arranged in the arrangement unit with an electron beam having an energy higher than 1 MeV to change the prodrug into an active substance. An electron beam irradiation method according to another aspect of the present invention includes a step of arranging an irradiation target containing a prodrug therein in an arrangement unit, and a step of irradiating the prodrug inside the irradiation target arranged in the arrangement unit with an electron beam having an energy higher than 1 MeV, to change the prodrug into an active substance.

In the electron beam irradiation device and the electron beam irradiation method, even in a case where the prodrug is present at the deep part inside the irradiation target, it is possible to selectively apply the electron beam to the prodrug at the sufficient dose to express the activity. Accordingly, the activity of the prodrug can be expressed in the deep part of the irradiation target.

The electron beam irradiation device according to the aspect of the present invention may include a first application unit configured to apply an external magnetic field along a traveling direction of the electron beam to the electron beam. In this case, it is possible to enhance the directivity of the electron beam, and it is possible to remarkably exhibit the above-described action and effect capable of selectively applying the electron beam with the sufficient dose to the prodrug present in the deep part inside the irradiation target.

In the electron beam irradiation device according to the aspect of the present invention, the irradiation unit may include an electron beam source configured to generate the electron beam having an energy chirp, and a chirp adjustment unit configured to adjust the energy chirp of the electron beam generated by the electron beam source such that the energy chirp is arranged at the rear in a beam traveling direction as an energy component becomes higher. In this case, as the electron beam propagates to the prodrug in the irradiation target, the pulse width is automatically gradually compressed and the charge density is increased, and it is possible to increase the energy application to the prodrug while suppressing the energy application to a part other than the prodrug in the irradiation target. That is, beam irradiation that selectively imparts high energy to the prodrug in the irradiation target can be realized by using the electron beam.

In the electron beam irradiation device according to the aspect of the present invention, the electron beam may have a pulse width of sub-picoseconds or less. In this case, the effect of the electric field of the electron beam on the prodrug is enhanced, and for example, the activity of the prodrug can be efficiently expressed as compared with a case where the electron beam has a pulse width in a range of nanoseconds to picoseconds.

The electron beam irradiation device according to the aspect of the present invention may include an input unit configured to be able to input prodrug information on the prodrug, and a control unit configured to control time structures of a plurality of electron pulses included in the electron beam emitted by the irradiation unit, based on the prodrug information. In this case, the time structures of the plurality of electron pulses are controlled based on the prodrug information, and thus, it is possible to control a chemical reaction due to an interaction between the electron pulse and the prodrug, and to control, for example, a structure, a type, a yield, a yield ratio, and the like of the active substance changed from the prodrug. The time structures of the plurality of electron pulses may include, for example, at least one of intensity, an intensity ratio, each time interval, and each pulse width for the plurality of electron pulses.

In the electron beam irradiation device according to the aspect of the present invention, the irradiation unit may emit the electron beam in which orientations of spins are aligned. In this case, for example, the structure, type, yield, yield ratio, and the like of the active substance changed from the prodrug can be controlled to the structure, type, yield, yield ratio, and the like corresponding to the orientations of the spins of the electron beam.

The electron beam irradiation device according to the aspect of the present invention may include a second application unit configured to apply an external electric field or an external magnetic field to the prodrug inside the irradiation target arranged in the arrangement unit such that orientations of polarities of the prodrug are aligned. In this case, for example, the structure, type, yield, yield ratio, and the like of the active substance changed from the prodrug can be controlled the structure, type, yield, yield ratio, and the like corresponding to the orientations of the polarities of the prodrug.

In the electron beam irradiation device according to the aspect of the present invention, the irradiation unit may be able to irradiate the prodrug inside the irradiation target arranged in the arrangement unit with the electron beam from a plurality of directions. In this case, the prodrug is irradiated with the electron beam from the plurality of directions, and thus, it is possible to enhance the energy application to the prodrug while suppressing the energy application to a part other than the prodrug in the irradiation target.

### Advantageous Effects of Invention

According to the aspect of the present invention, it is possible to provide the electron beam irradiation device and the electron beam irradiation method capable of expressing the activity of the prodrug even in the deep part of the irradiation target.

### Brief Description of Drawings

FIG. 1 is a schematic configuration diagram illustrating an electron beam irradiation device according to a first embodiment.
FIG. 2(a) is a schematic diagram illustrating a phase rotator. FIG. 2(b) is a schematic diagram illustrating a path adjuster.
FIG. 3 is a graph representing an example of a time structure of an electron beam.
FIG. 4 is a diagram illustrating an example of a dose distribution of the electron beam.
FIG. 5 is a graph representing a relationship between a relative dose of the electron beam and a depth of an irradiation target.
FIG. 6(a) is a schematic diagram for explaining a relationship between a pulse width of the electron beam and a shielding effect. FIG. 6(b) is another schematic diagram for explaining the relationship between the pulse width of the electron beam and the shielding effect.
FIG. 7 is a schematic configuration diagram illustrating an electron beam irradiation device according to a second embodiment.
FIG. 8(a) is a schematic configuration diagram illustrating an irradiation unit according to a modification example. FIG. 8(b) is a schematic configuration diagram illustrating an irradiation unit according to another modification example.

### Description of Embodiments

Hereinafter, embodiments will be described in detail with reference to the drawings. In the following description, the same or equivalent components are denoted by the same reference signs, and the redundant description will be omitted.

### [First Embodiment]

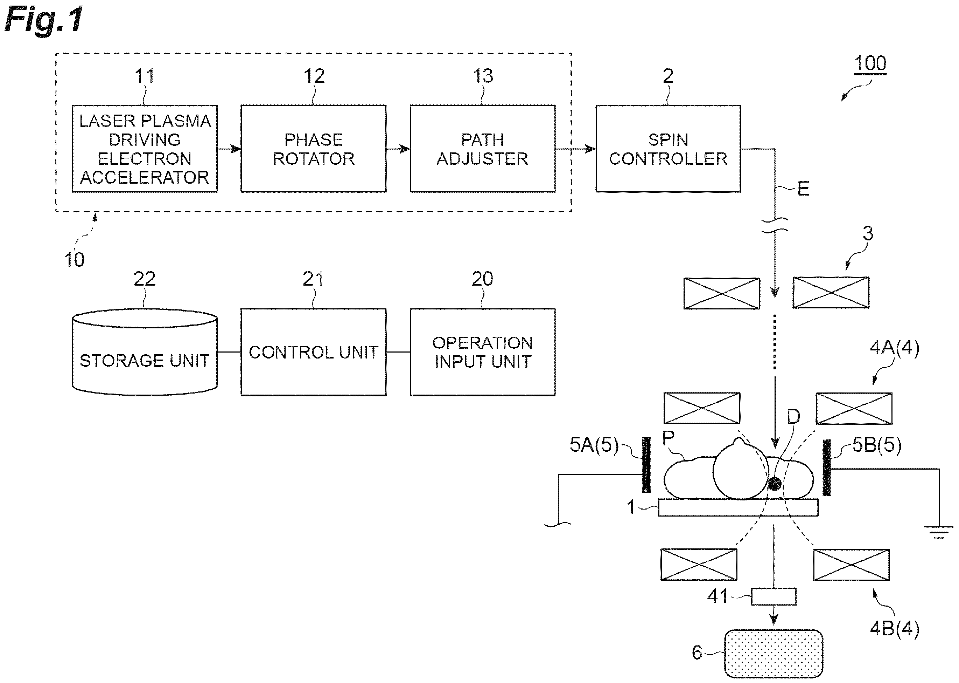

As illustrated in FIG. 1, an electron beam irradiation device 100 according to a first embodiment is a device that irradiates a prodrug D in a body of a patient (irradiation target) P with an electron beam E and changes the prodrug D into an active substance by causing the electron beam E to act as a trigger. The electron beam irradiation device 100 includes a patient table 1, an irradiation unit 10, a spin controller 2, a first magnetic field application unit 3, a second magnetic field application unit 4, an electric field application unit 5, a beam monitor 41, a beam dump 6, an operation input unit 20, and a control unit 21.

The prodrug D is a drug that is changed to the active substance (expresses activity) by a trigger after reaching a target site in the body of a patient P. The prodrug D can express activity and function at an aimed part. The prodrug D is not particularly limited, and may be any prodrug. For example, as the prodrug D, a drug that is inactive until reaching the target site and can be changed to a drug such as an anticancer agent by a trigger is used at the target site. The prodrug D may be present at a deep position (body trunk) in the body of the patient P.

The patient table 1 is an arrangement unit in which the patient P containing the prodrug D therein is placed. The irradiation unit 10 is a unit that irradiates the prodrug D inside the patient P on the patient table 1 with the electron beam E to change the prodrug D into the active substance. The irradiation unit 10 includes a laser plasma driving electron accelerator 11, a phase rotator 12, and a path adjuster 13.

The laser plasma driving electron accelerator 11 is an electron accelerator that accelerates an electron beam by laser plasma acceleration. An acceleration energy of the laser plasma driving electron accelerator 11 is, for example, 1 to 300 Me V. The laser plasma driving electron accelerator 11 constitutes an electron beam source that emits the electron beam E of an ultrashort electron pulse with a wide energy range. The laser plasma driving electron accelerator 11 includes an optical waveguide forming apparatus that forms, for example, an optical waveguide that propagates a high-intensity laser pulse over a long distance by gas discharge in a discharge tube.

The electron beam E generated by the laser plasma driving electron accelerator 11 has an energy chirp due to expansion of an energy spectrum by crushing of a plasma wave. The energy chirp means a state where an energy component of the electron beam E has a strong correlation with a traveling direction, and a state where the energy component gradually changes from the front to the rear in the traveling direction of the electron beam E. The electron beam E generated by the laser plasma driving electron accelerator 11 has, for example, a ΔE/E of 100 to 0.1%. E mentioned herein is an acceleration energy, and ΔE is an energy width (spectrum width). ΔE corresponds to, for example, a half width of a distribution of the acceleration energy. For example, ΔE/E = 10 MeV/100 MeV = 10%.

The phase rotator 12 is a chirp adjustment unit that spatially rotates the electron beam E by 180° and inverts the energy chirp of the electron beam E. The phase rotator 12 adjusts the energy chirp of the electron beam E such that the energy chirp is arranged at the rear in the beam traveling direction as the energy component becomes higher (in other words, the energy chirp is arranged in front of the beam traveling direction as the energy component becomes lower). An electronic bunch stretcher or the like can be used as the phase rotator 12.

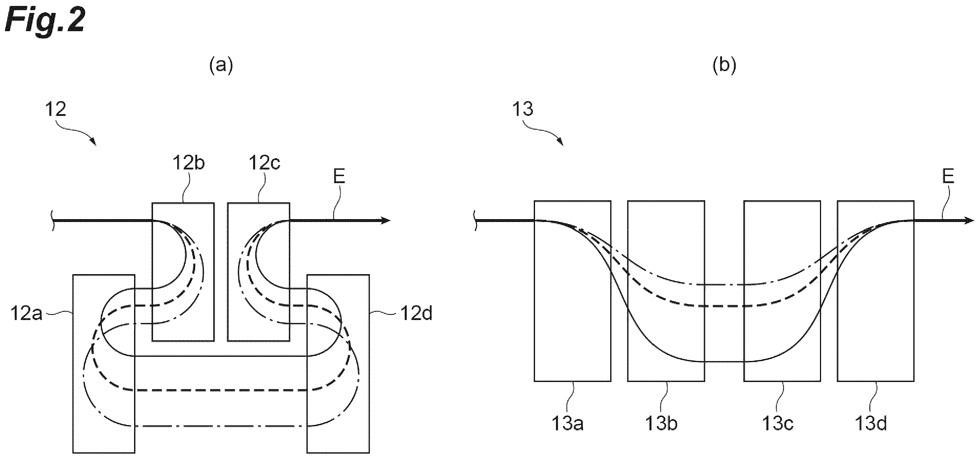

For example, as illustrated in FIG. 2(a), the phase rotator 12 includes magnetic field forming units 12a, 12b, 12c, and 12d constituted by dipole magnets. In the phase rotator 12, electrons move to form a semicircular orbit by magnetic fields of the magnetic field forming units 12a, 12b, 12c, and 12d. As a result, the emitted electron beam E has the energy chirp arranged at the rear in the beam traveling direction as the energy component becomes higher.

The path adjuster 13 compresses a pulse width of the electron beam E adjusted by the phase rotator 12. The path adjuster 13 adjusts an optical path of each energy component of the electron beam E and compresses the pulse width of the electron beam E. The path adjuster 13 compresses the electron beam E in a longitudinal direction (direction along the traveling direction of the electron beam E). The path adjuster 13 adjusts the optical path of each energy component of the electron beam E such that the pulse width of the electron beam E is most compressed at a position of the prodrug D. An electronic bunch compressor or the like can be used as the phase rotator 12.

For example, as illustrated in FIG. 2(b), the path adjuster 13 includes electromagnets 13a, 13b, 13c, and 13d. In the path adjuster 13, the electrons move to form an arc-shaped orbit by the magnetic fields of the electromagnets 13a, 13b, 13c, and 13d. As a result, the optical path of each energy component of the electron beam E is adjusted, and an optical path distance from the path adjuster 13 to the prodrug D is set to a distance at which the pulse width of the electron beam E is most compressed at the position of the prodrug D.

The electron beam E emitted from the irradiation unit 10 having the above configuration has the following characteristics, for example. That is, the electron beam E has a pulse width of sub-picoseconds or less. Here, the electron beam E has a pulse width of 300 femtoseconds or less. Specifically, the electron beam E has a pulse width of 100 femtoseconds or less, and more specifically, has a pulse width of several femtoseconds or less. The electron beam E has an energy that can pass through the patient P and reach the prodrug D. The electron beam E has an energy higher than 1 MeV, here higher than 10 MeV, specifically higher than 200 MeV. Each condition of the electron beam E may be settable via the operation input unit 20, for example. In a case where the irradiation target is the patient P as in the present embodiment, the electron beam E may have an energy higher than 10 MeV.

Referring back to FIG. 1, the spin controller 2 controls spins of the electron beam E emitted from the irradiation unit 10 to be aligned in a specific direction. The spin controller 2 spin-polarizes the electron beam E input from the irradiation unit 10 and emits the electron beam E with aligned orientations of the spins. Here, the spin controller 2 aligns spins in directions corresponding to polarities of the prodrug D aligned by the electric field application unit 5. The spin controller 2 is not particularly limited, and various controllers may be used. The directions of the spins to be aligned by the spin controller 2 may be fixed or variable. In a case where the directions of the spins to be aligned by the spin controller 2 are variable, for example, an angle may be changeable via the operation input unit 20.

The first magnetic field application unit 3 applies an external magnetic field along the traveling direction (propagation direction) of the electron beam E to the electron beam E input from the spin controller 2. The first magnetic field application unit 3 focuses the electron beam E. The first magnetic field application unit 3 compresses the electron beam E in a lateral direction (direction perpendicular to the traveling direction). For example, an electromagnet driven by a pulse current in time synchronization with the electron beam E is used as the first magnetic field application unit 3. The first magnetic field application unit 3 constitutes a first application unit. The first magnetic field application unit 3 is not limited, and a superconducting magnet or the like may be used.

The second magnetic field application unit 4 applies an external magnetic field along the traveling direction of the electron beam E to the electron beam E with which the prodrug D in the body of the patient P on the patient table 1 is irradiated. The second magnetic field application unit 4 includes an upstream application unit 4A arranged on an upstream side of the patient table 1 in the traveling direction of the electron beam E and a downstream application unit 4B arranged on a downstream side of the patient table 1 in the traveling direction of the electron beam. For example, an electromagnet driven by a pulse current in time synchronization with the electron beam E is used as the upstream application unit 4A and the downstream application unit 4B. The second magnetic field application unit 4 focuses the electron beam E between the upstream application unit 4A and the downstream application unit 4B. The second magnetic field application unit 4 compresses the electron beam E in the lateral direction between the upstream application unit 4A and the downstream application unit 4B. The second magnetic field application unit 4 constitutes the first application unit. The second magnetic field application unit 4 is not limited, and a superconducting magnet or the like may be used.

The electric field application unit 5 applies an external electric field to the prodrug D inside the patient P arranged on the patient table 1 such that orientations of the polarities of the prodrug D are aligned. In the electric field application unit 5, electrodes 5A and 5B are provided to sandwich the patient P. A direction in which the electrodes 5A and 5B of the electric field application unit 5 face each other is orthogonal to the traveling direction of the electron beam E. The orientations of the polarities aligned by the electric field application unit 5 may be fixed or variable. In a case where the orientations of the polarities to be aligned by the electric field application unit 5 are variable, for example, an angle may be changeable via the operation input unit 20. The orientations of the polarities aligned by the electric field application unit 5 may be an orientation corresponding to the directions of the spins aligned by the spin controller 2. The electric field application unit 5 constitutes a second application unit.

The beam monitor 41 is a device that measures the electron beam E having transmitted through the prodrug D. The beam monitor 41 monitors at least one of a position, intensity, a shape, and dose of the electron beam E having transmitted through the prodrug D. The beam monitor 41 is not particularly limited, and a known monitor can be used. A beam dump 6 is a device that absorbs and stops the electron beam E having transmitted through the prodrug D and measured by the beam monitor 41. The beam dump 6 is provided on the downstream side of the patient table 1 in the optical path of the electron beam E. The beam dump 6 is not particularly limited, and a known device can be used.

The operation input unit 20 is an input unit capable of receiving various inputs by the user. For example, a touch panel, a microphone, a camera, and the like are used as the operation input unit 20. Examples of the input of the operation input unit 20 include a touch input, a voice input, a camera input, and the like. The operation input unit 20 is configured to be able to input, for example, each condition of the electron beam E emitted from the irradiation unit 10, the directions of the spins to be aligned by the spin controller 2, the orientations of the polarities to be aligned by the electric field application unit 5, and prodrug information related to the prodrug D. The prodrug information includes at least information on a type of the prodrug D.

The control unit 21 includes, for example, one or more computer devices. The control unit 21 includes a central processing unit (CPU) that is a processor, a random access memory (RAM) or a read only memory (ROM) that is a recording medium, and the like. The control unit 21 executes various kinds of control by loading a program or the like on hardware such as the CPU and the RAM. The control unit 21 controls each condition of the electron beam E emitted by the irradiation unit 10 based on the input of the operation input unit 20.

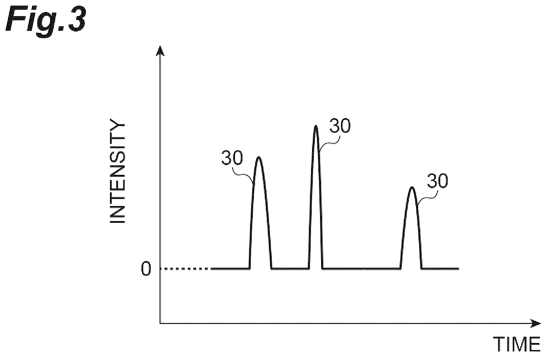

The control unit 21 controls a time structure of a plurality of electron pulses included in the electron beam E emitted by the irradiation unit 10, based on the prodrug information input by the operation input unit 20. Specifically, the control unit 21 controls the time structures of the electron pulses included in the electron beam E while referring to a data table stored in the storage unit 22 from the type of the prodrug D in the input prodrug information. For example, as illustrated in FIG. 3, the time structure of the electron pulse includes at least one of intensity, an intensity ratio, each time interval, and each pulse width for the plurality of electron pulses 30. For example, in the data table, the type of the prodrug D and the time structure of the electron pulse are associated with each other. The data table can be acquired in advance by actual measurement, simulation, or the like.

In a case where the electron beam irradiation device 100 described above is used, first, the patient P having the prodrug D reached the target site in the body is arranged on the patient table 1. At the same time, each condition of the electron beam E to be emitted, the prodrug information, the spin orientation of the electron beam E, and orientations of polarities of the prodrug D are input by the operation input unit 20. As a result, the irradiation unit 10 is controlled by the control unit 21, and for example, the electron beam E having the pulse width of 100 femtoseconds or less with the energy greater than 200 MeV and having the time structure of the electron pulse corresponding to the type of the prodrug D is emitted from the irradiation unit 10.

After the directions of the spins of the electron beam E emitted from the irradiation unit 10 are aligned by the spin controller 2 and the electron beam is focused by the first magnetic field application unit 3, the electron beam E is selectively emitted to the prodrug D in the body of the patient P placed on the patient table 1. At this time, the electron beam E is focused by the second magnetic field application unit 4. In addition, the orientations of the polarities of the prodrug D are aligned by the electric field application unit 5. As a result, the prodrug D is activated by irradiation with the electron beam E, is changed to the active substance, and expresses the activity thereof.

As a result, in the present embodiment, since the high energy electron beam E has low energy attenuation in the substance, the electron beam E can sufficiently reach the prodrug D present in a deep part of the patient P. Thus, even in a case where the prodrug D is present at the deep part inside the patient P, the electron beam E can be selectively applied to the prodrug D with a sufficient dose, and the electron beam E can act as the trigger. As a result, it is possible to activate the prodrug D to change the prodrug to the active substance, and it is possible to express the activity thereof.

That is, according to the electron beam irradiation device 100 and an electron beam irradiation method, it is possible to express the activity of the prodrug D in the deep part of the patient P. The irradiation of the electron beam E capable of stereoscopically controlling the energy application to the body of the patient P is used, and thus, the activity and function of the prodrug D can be expressed in the deep part of the body trunk.

The electron beam irradiation device 100 includes the first magnetic field application unit 3 and the second magnetic field application unit 4. In this case, directivity of the electron beam E can be enhanced, and the above-described action and effect that can selectively apply the electron beam E having a pencil beam shape with a sufficient dose to the prodrug D present in a deep part inside the patient P can be remarkably exhibited. A problem that even the high energy electron beam E is scattered in the substance is suppressed, and the directivity of the electron beam E can be maintained. The prodrug D localized by a drug delivery system is irradiated with the aggregated electron beam E, and thus, the prodrug D can be efficiently activated while reducing the influence on the human body.

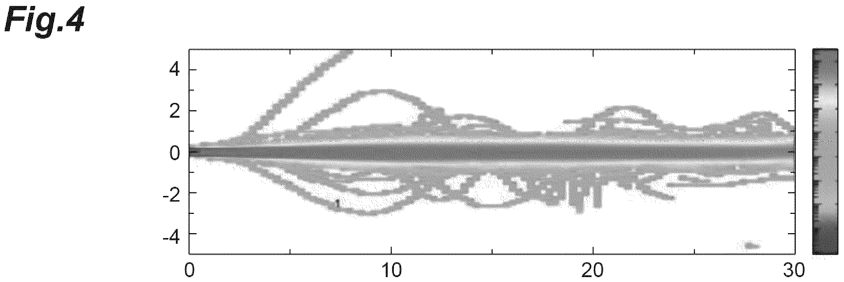

FIG. 4 is a diagram illustrating an example of a dose distribution of the electron beam E. FIG. 4 is a numerical simulation result illustrating a state where the electron beam E having an energy of 200 MeV propagates in water. In FIG. 4, a horizontal axis represents a position in the traveling direction of the electron beam E, and a vertical axis represents a position in a direction perpendicular to the traveling direction. In a numerical simulation in the drawing, a magnetic field of 3 T is applied in the traveling direction of the electron beam E. The denser the shading in the drawing, the larger the dose (flux) of the electron beam E. As illustrated in FIG. 4, in the present embodiment, it can be seen that the diffusion of the electron beam E is suppressed and the electron beam E is focused in the pencil beam shape to enhance the directivity.

In the electron beam irradiation device 100, the irradiation unit 10 includes the laser plasma driving electron accelerator 11, the phase rotator 12, and the path adjuster 13. In this case, with the propagation of the electron beam E to the prodrug D in the body of the patient P, the pulse width is automatically gradually compressed to increase a charge density, and it is possible to increase the energy application to the prodrug D while suppressing the energy application to a part other than the prodrug D in the patient P. That is, beam irradiation that selectively applies a large energy to the prodrug D in the body of the patient P can be realized by using the electron beam E. In addition, the first magnetic field application unit 3 and the second magnetic field application unit 4 are combined, and thus, three-dimensional irradiation control of the electron beam E can be easily performed. The prodrug D localized by a drug delivery system is irradiated with the aggregated electron beam E, and thus, the prodrug D can be efficiently activated while reducing the influence on the human body.

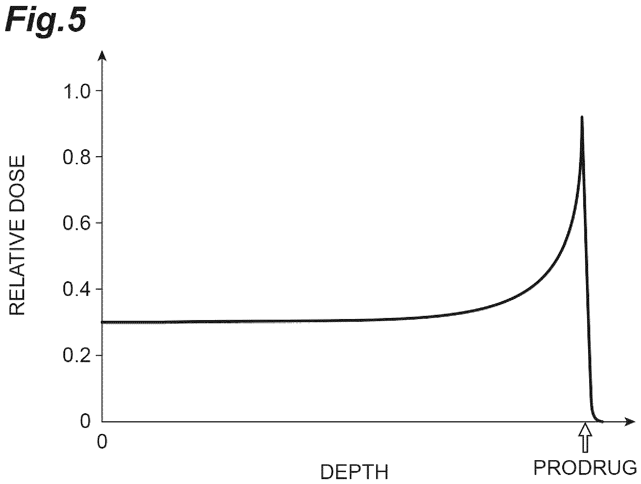

FIG. 5 is a graph representing a dose application distribution of the electron beam E. A vertical axis in the drawing represents a relative dose of the electron beam E, and a horizontal axis in the drawing represents a depth from an electron beam incident surface of the patient P. As illustrated in FIG. 5, in a depth region up to the prodrug D, the relative dose of the electron beam E is maintained in a low state, whereas the relative dose of the electron beam E steeply increases at a depth position of the prodrug D. As described above, in the present embodiment, it can be expected that irradiation similar to Bragg peak characteristics of a Hadron beam is performed with the electron beam even though the electron beam E is used.

In the electron beam irradiation device 100, the electron beam E has the pulse width of sub-picoseconds or less. In this case, the effect of the electric field of the electron beam E on the prodrug D is enhanced, and for example, as compared with a case where the electron beam E has a pulse width in a range of nanoseconds to picoseconds, the activity of the prodrug D can be efficiently expressed (that is, change from the prodrug D to the active substance with a small total dose), and an exposure dose of the patient P can be reduced.

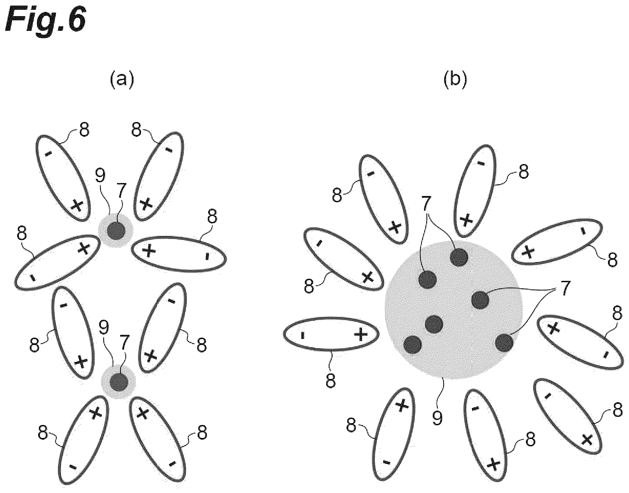

FIG. 6 is a schematic diagram for explaining a relationship between the pulse width of the electron beam E and a shielding effect. As illustrated in FIG. 6(a), generally, when the electron beam E propagates in a condensed dipole medium (water), electrons 7 in the electron beam E are shielded by water molecules 8. Thus, an electric field 9 generated by the electrons 7 is small, and does not influence the motion and dynamics of the electron beam E. However, polarization reaction requires a finite time, and for example, assuming that a rotation speed v of a dipole is 10⁵ cm/s and a length l of the dipole is 3 × 10⁻⁸ cm, a relaxation time TR is 300 fs (3 × 10⁻¹³ s: femtosecond). When the pulse width of the electron beam E is shorter than the relaxation time, the water molecules 8 shield the electric field 9 only on a surface of the electron beam E as illustrated in FIG. 6(b). Thus, a region of the strong electric field 9 is formed. From this, it is significant that the electron beam E has the pulse width of sub-picoseconds or less.

The electron beam irradiation device 100 includes the operation input unit 20 and the control unit 21. In this case, the time structure (for example, intensity, intensity ratio, time interval, pulse width, and the like) of the plurality of electron pulses 30 is controlled by the control unit 21 based on the prodrug information input via the operation input unit 20. As a result, it is possible to control chemical reaction due to an interaction between the electron pulse 30 and the prodrug D and to control, for example, a structure, a type, a yield, a yield ratio, and the like of the active substance changed from the prodrug D.

In the electron beam irradiation device 100, the orientations of the spins of the electron beam E from the irradiation unit 10 are aligned by the spin controller 2. In this case, for example, the structure, type, yield, yield ratio, and the like of the active substance changed from the prodrug D can be controlled to the structure, type, yield, yield ratio, and the like corresponding to the aligned orientations of the spins of the electron beam E. Note that, the spin controller 2 may align the orientations of the spins in accordance with the orientations of the polarities of the prodrug D aligned by the electric field application unit 5, or may align the orientations of the spins independently.

The electron beam irradiation device 100 includes the electric field application unit 5. In this case, for example, the structure, type, yield, yield ratio, and the like of the active substance changed from the prodrug D can be controlled to the structure, type, yield, yield ratio, and the like corresponding to the aligned orientations of the polarities of the prodrug D. Note that, the electric field application unit 5 may align the orientations of the polarities in accordance with the orientations of the spins of the electron beam E aligned by the spin controller 2, or may align the orientations of the polarities independently.

The activation of the prodrug D (the generation of the anticancer agent) by irradiation with the electron beam E was evaluated and tested under the following conditions by using the electron beam irradiation device 100 and the electron beam irradiation method according to the present embodiment.
PBS (100mM, pH = 7, DMSO (0.1%)
Energy of electron beam E: 11.5 Gy/1 shot
Reverse layer HPLC
Column: ODS
Mobile phase: water, acetonitrile, formic acid 0.1%

As a result, a peak of a generation amount was observed at a retention time of 3.9 minutes by irradiation with the electron beam E. The generation of the anticancer agent could be confirmed. It could be confirmed that 5% of the anticancer agent was generated in one irradiation.

In the above description, the irradiation unit 10 and the spin controller 2 constitute an irradiation unit. The first magnetic field application unit 3 and the second magnetic field application unit 4 constitute a magnetic field application unit. At least one of the phase rotator 12, the path adjuster 13, the spin controller 2, the first magnetic field application unit 3, the second magnetic field application unit 4, and the electric field application unit 5 may be omitted in some cases.

Note that, in the present embodiment, a positional relationship between the patient table 1 and the irradiation unit 10 may be configured to be controllable by the control unit 21 such that the electron beam E from the irradiation unit 10 is appropriately incident on the prodrug D in the body of the patient P on the patient table 1. In addition, for example, the operation input unit 20 may be configured to be able to input positional information of the prodrug D in the body of the patient P. In this case, the positional relationship between the patient table 1 and the irradiation unit 10 may be configured to be controllable by the control unit 21 such that the electron beam E is incident on the prodrug D based on the positional information. In addition, for example, the electric field application unit 5 may be provided such that a relative positional relationship with respect to the patient table 1 can be changed. As a result, an external electric field can be easily applied toward the prodrug D inside the patient P on the patient table 1.

The present embodiment includes the following electron beam irradiation method.

The electron beam irradiation method includes a step (arrangement step) of arranging the irradiation target containing the prodrug therein in an arrangement unit, and a step (irradiating step) of irradiating the prodrug inside the irradiation target arranged in the arrangement unit with the electron beam having the energy higher than 1 MeV to change the prodrug into the active substance. The electron beam irradiation method corresponds to a method for using the electron beam irradiation device 100.

The electron beam irradiation method includes a magnetic field application step of applying the external magnetic field along the traveling direction of the electron beam to the electron beam. In the electron beam irradiation method, the irradiation step includes a step of generating the electron beam having the energy chirp, and a step of adjusting the energy chirp of the generated electron beam such that the higher energy component is arranged in the rear in the beam traveling direction.

In the electron beam irradiation method, the electron beam has the pulse width of sub-picoseconds or less. The electron beam irradiation method includes an input step of inputting the prodrug information regarding the prodrug, and a control step of controlling the time structure of the plurality of electron pulses 30 included in the irradiated electron beam, based on the prodrug information. In the electron beam irradiation method, in the irradiation step, the electron beam in which the orientations of the spins are aligned is emitted.

The electron beam irradiation method includes an electric field application step of applying the external electric field to the prodrug inside the irradiation target arranged in the arrangement unit such that the orientations of the polarities of the prodrug are aligned. In the electron beam irradiation method, in the irradiation step, the prodrug inside the arranged irradiation target is irradiated with the electron beam from the plurality of directions.

The present embodiment may be used for simultaneous activation of a plurality of prodrugs. In cancer therapy, multi-drug combination therapy using a plurality of anticancer agents in combination is often performed, but in this case, toxicity of each anticancer agent is a problem. In the present embodiment, it is also expected to increase a therapy satisfaction level of the multi-drug combination therapy by prodrug conversion (inactivation) of each anticancer agent to be used in combination and simultaneous activation by electron beam irradiation (multi-prodrug therapy). Further, the present embodiment may be used for a molecule (dual prodrug therapy) activated by a plurality of prodrugs D by linking a plurality of anticancer agents to one molecule via a linker or the like.

In the present embodiment, the prodrug may include a drug carrier in a broad sense. The drug carrier contains a drug, and examples thereof include polymeric micelles, liposomes, and nanomachines. That is, in one aspect of the present invention, it is also possible to use for site-specific disintegration and drug release of the drug carrier. The drug carrier accumulated in an affected area, such as cancer tissue, is irradiated with the high energy electron beam, and thus, disintegration of the drug carrier selectively in the affected area and subsequent drug release are expected.

Reaction (reaction unique to the electron beam E) when the prodrug is irradiated with the electron beam E as the trigger will be specifically described. In a case where the prodrug D is irradiated with the electron beam E as the trigger, hydrated electrons are generated from water molecules by high energy electron beam irradiation as a first stage (see the following formula).

H₂O → H₂O⁺ + e_{aq}⁻ (initial step)

As a second stage, in a prodrug (Inactive) in which a drug and a mask are bound, hydrated electrons react with the mask, and the drug is released. A specific masking group is an azide group (phenyl azide, alkyl azide, and sulfone azide).

In addition, in particular, in a case where a drug carrier as a prodrug is irradiated with the electron beam E as the trigger, hydrated electrons are generated from water molecules by high energy electron beam irradiation as the first stage (see the following formula).

H₂O → H₂O⁺ + e_{aq}⁻ (initial step)

As a second stage, hydrated electrons act on amphiphilic molecules constituting the drug carrier to change a chemical structure. After the drug carrier is disintegrated, and the drug (drug-encapsulating core) is released. In an example in which hydrated electrons react with such amphiphilic molecules, it is considered that a required dose is high as compared with the above-described example in which the hydrated electrons react with the mask.

The present embodiment may be used for therapy of neurodegenerative diseases such as Alzheimer's disease and brain diseases such as brain tumors. In a case where the brain diseases are targeted, an administered drug needs to transit from the blood into the brain, but, in this case, the administered drug needs to across a blood-brain barrier separating the blood and brain tissue. In general, it is known that a drug having high lipid solubility has high intracerebral migration. In the present embodiment, a substituent is masked with low lipid solubility in a drug, and thus, the prodrug D with improved lipid solubility is produced. When the prodrug D reaches the brain and then is activated by irradiation with the electron beam E, it is expected that the intracerebral migration can be improved and the prodrug D can act specifically on the brain.

The present embodiment can be applied to the drug delivery system. In the drug delivery system, it is possible to deliver the drug to a desired part by utilizing an enhanced permeability (EPR) effect. That is, a blood vessel has a gap for taking oxygen or nutrients into cells outside the blood vessel. In normal blood vessels, this gap is very small, small molecules can pass through a vessel wall but cannot pass through macromolecules. A blood vessel of a tumor tissue has many branches since the blood vessel is formed by angiogenesis associated with growth of a tumor, and a blood vessel wall is coarser than a normal blood vessel, and a gap of, for example, about 100 to 200 nm is formed. This difference is used, and thus, the drug carrier can allow the drug to accumulate in the tumor tissue.

In addition, in the drug delivery system, the drug can be delivered to the desired part by using an antibody-drug conjugate (ADC). That is, a molecule in which an antibody and a drug are linked via a linker is referred to as an antibody-drug conjugate (ADC). The antibody is used as a precise recognition and delivery functional site of the target, and the drug is responsible for substantial drug efficacy. Thus, when the ADC is used, the drug can be specifically delivered to the cell recognized by the antibody to be used.

### [Second Embodiment]

Next, a second embodiment will be described. In the description of the present embodiment, points different from the first embodiment will be mainly described, and redundant description will be omitted.

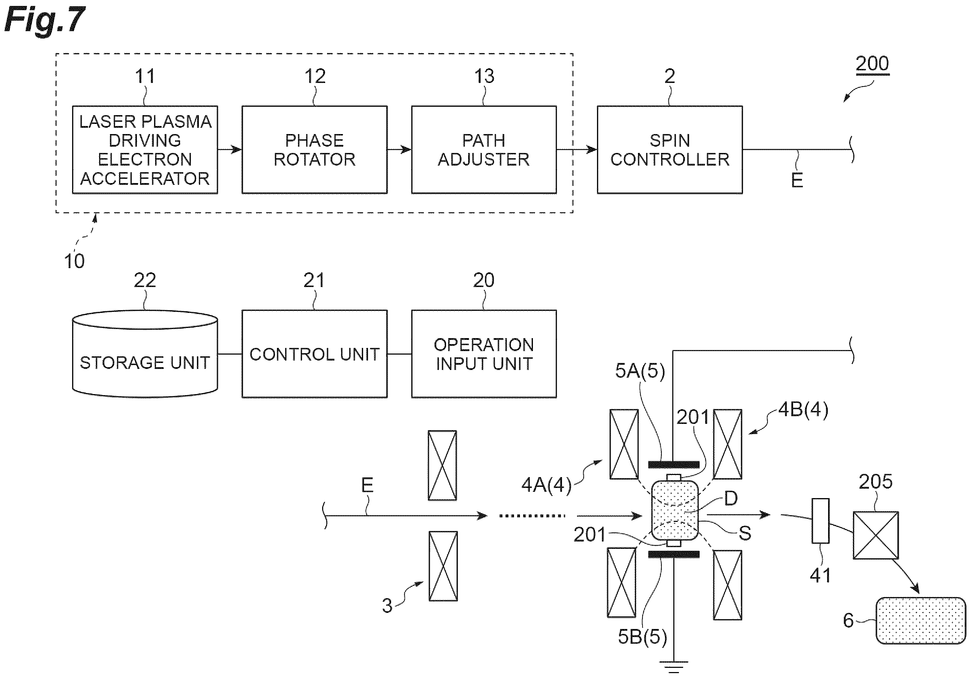

As illustrated in FIG. 7, an electron beam irradiation device 200 according to a second embodiment is a drug discovery platform used for development of the prodrug D. The electron beam irradiation device 200 is a device that irradiates the prodrug D inside a test tube (irradiation target) S with an electron beam E and causes the electron beam E to act as a trigger to change the prodrug D into an active substance.

The electron beam irradiation device 200 is different from the first embodiment in including a test tube holding unit 201 instead of the patient table 1 (see FIG. 1) and further including a beam deflection magnet 205. The test tube holding unit 201 holds and arranges a test tube S containing the prodrug D therein. The beam deflection magnet 205 is arranged between the beam monitor 41 and the beam dump 6. The beam deflection magnet 205 transmits the prodrug D and deflects the electron beam E measured by the beam monitor 41 toward the beam dump 6. The electron beam E of the present embodiment has an energy that can pass through the test tube S and reach the prodrug D. In the drug discovery platform as in the present embodiment (for example, when the irradiation target is the test tube S), the electron beam E may have an energy higher than 1 MeV.

As described above, in the electron beam irradiation device 200, an effect similar to the first embodiment, that is, an effect of making it possible to express the activity of the prodrug D in the deep part of the patient P is also exhibited. In the present embodiment, the beam deflection magnet 205 may be omitted in some cases.

[Modification Examples] Although the embodiments have been described above, one aspect of the present invention is not limited to the above embodiments. For example, each numerical value in the above and drawings may include an error in design, measurement, manufacturing, or the like.

The above embodiments may further include a phantom (water cell) arranged between the irradiation unit 10 and the patient P (preferably, in front of the body of the patient P) on the optical path of the electron beam E. This phantom functions similarly to the path adjuster 13, and can be adjusted such that a compression point of the electron beam E becomes the prodrug D in the body of the patient P. In the above embodiments, the irradiation target is not particularly limited, and may be various targets.

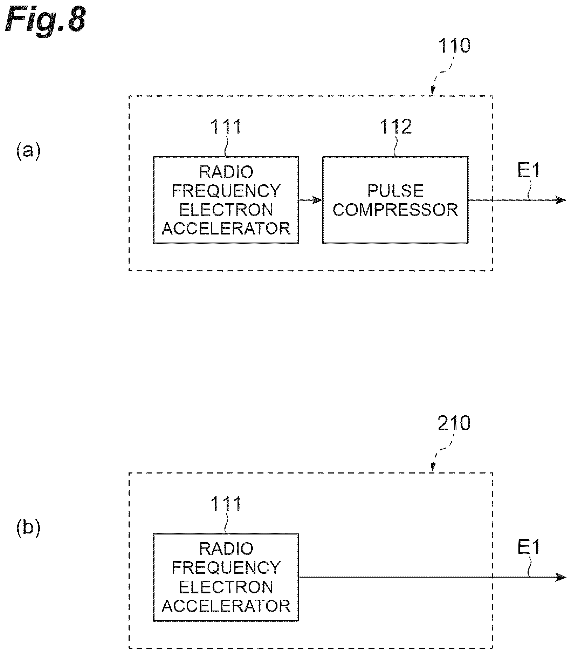

The above embodiments may include an irradiation unit 110 illustrated in FIG. 8(a) instead of the irradiation unit 10 (see FIG. 1). As illustrated in FIG. 8(a), the irradiation unit 110 includes a radio frequency electron accelerator 111 and a pulse compressor 112. The radio frequency electron accelerator 111 emits an electron beam E1 of a monochromatic (single energy) electron pulse. The pulse compressor 112 compresses a pulse of the electron beam E1 emitted by the radio frequency electron accelerator 111 and outputs the pulse to the spin controller 2 (see FIG. 1) at a subsequent stage. For example, the pulse compressor 112 compresses the pulse width of the electron beam E1 to several nanoseconds to several femtoseconds. Note that, the pulse compressor 112 may be omitted in some cases (see an irradiation unit 210 in FIG. 8(b)). The irradiation unit 110 or 210 constitutes an irradiation unit.

In the above embodiments, although an example in which the prodrug D is irradiated with the electron beam E from a specific irradiation direction has been described, the present invention is not limited thereto. The irradiation unit may be capable of irradiating the prodrug inside the irradiation target arranged in the arrangement unit with the electron beam from the plurality of directions. For example, an emission side of the irradiation unit 10 is configured to be movable around the irradiation target, and the irradiation of the prodrug D whose position on the emission side of the irradiation unit 10 has been changed with the electron beam E may be performed multiple times. In this case, the prodrug D is irradiated with the intensity-modulated electron beam E from multiple directions and superimposing the electron beam E at the position of the prodrug D, and thus, it is possible to suppress the energy application to a part other than the prodrug D in the irradiation target and enhance the energy application to the prodrug D.

In the above embodiments, in a case where the spins of the electron beam E emitted from the irradiation unit 10 are aligned, the spin controller 2 may not be provided. In a case where the spin controller 2 is provided, the spins of the electron beam E can be easily and reliably controlled and aligned by the spin controller 2. In the above embodiments, the electric field application unit 5 is provided as the second application unit, but in place of or in addition to this, a magnetic field application unit (for example, a magnetic resonance imaging (MRI) device) that applies an external magnetic field such that the orientations of the polarities of the prodrug D are aligned with respect to the prodrug D may be provided as the second application unit.

The above embodiments may further include a phantom (water cell) arranged between the phase rotator 12 and the irradiation target (preferably, in front of the irradiation target) on the optical path of the electron beam E. The phantom functions as an electron beam compression unit similarly to the path adjuster 13, and can be adjusted such that a compression point of the electron beam E becomes the prodrug D inside the irradiation target. In addition, in the above embodiments, the patient P and the test tube S are irradiated with the electron beam E, but the irradiation target is not particularly limited.

In the above embodiments, the external electric field and the external magnetic field for aligning the orientations of the polarities of the prodrug D can be generated and applied by various known techniques.

### Reference Signs List

- 1: patient table (arrangement unit)
- 2: spin controller (irradiation unit)
- 3: first magnetic field application unit (first application unit)
- 4: second magnetic field application unit (first application unit)
- 5: electric field application unit (second application unit)
- 10, 110, 210: irradiation unit (irradiation unit)
- 11: laser plasma driving electron accelerator (electron beam source)
- 12: phase rotator (chirp adjustment unit)
- 20: operation input unit (input unit)
- 21: control unit
- 100, 200: electron beam irradiation device
- 201: test tube holding table (arrangement unit)
- D: prodrug
- E, E1: electron beam
- P: patient (irradiation target)
- S: test tube (irradiation target)

## Claims

1. An electron beam irradiation device comprising:
an arrangement unit configured to arrange an irradiation target containing a prodrug therein; and
an irradiation unit configured to irradiate the prodrug inside the irradiation target arranged in the arrangement unit with an electron beam having an energy higher than 1 MeV, to change the prodrug into an active substance.

2. The electron beam irradiation device according to claim 1, further comprising a first application unit configured to apply an external magnetic field along a traveling direction of the electron beam to the electron beam.

3. The electron beam irradiation device according to claim 1 or 2, wherein
the irradiation unit includes
an electron beam source configured to generate the electron beam having an energy chirp, and
a chirp adjustment unit configured to adjust the energy chirp of the electron beam generated by the electron beam source such that the energy chirp is arranged at a rear in a beam traveling direction as an energy component becomes higher.

4. The electron beam irradiation device according to claim 1, wherein the electron beam has a pulse width of sub-picoseconds or less.

5. The electron beam irradiation device according to claim 1, further comprising:
an input unit configured to be able to input prodrug information on the prodrug; and
a control unit configured to control time structures of a plurality of electron pulses included in the electron beam emitted by the irradiation unit, based on the prodrug information.

6. The electron beam irradiation device according to claim 1, wherein the irradiation unit emits the electron beam in which orientations of spins are aligned.

7. The electron beam irradiation device according to claim 1 or 6, further comprising a second application unit configured to apply an external electric field or an external magnetic field to the prodrug inside the irradiation target arranged in the arrangement unit such that orientations of polarities of the prodrug are aligned.

8. The electron beam irradiation device according to claim 1, wherein the irradiation unit is able to irradiate the prodrug inside the irradiation target arranged in the arrangement unit with the electron beam from a plurality of directions.

9. An electron beam irradiation method comprising:
a step of arranging an irradiation target containing a prodrug therein in an arrangement unit; and
a step of irradiating the prodrug inside the irradiation target arranged in the arrangement unit with an electron beam having an energy higher than 1 MeV, to change the prodrug into an active substance.
